(19) Europäisches Patentamt
European Patent Office
Office européen des brevets

(11) Publication number: 0 132 812
A1

# (12) EUROPEAN PATENT APPLICATION

(21) Application number: 84108643.2

(22) Date of filing: 21.07.84

(51) Int. Cl.[4]: G 01 N 33/533

(30) Priority: 25.07.83 US 517236

(43) Date of publication of application:
13.02.85 Bulletin 85/7

(84) Designated Contracting States:
BE DE FR GB IT NL SE

(71) Applicant: Becton Dickinson and Company
Mack Centre Drive
Paramus New Jersey 07652(US)

(72) Inventor: Wagner, Daniel B.
7705 Moorgate Court
Raleigh North Carolina 27609(US)

(72) Inventor: Tyson, Richard L.
1412 Lake Park Drive
Raleigh North Carolina 27612(US)

(74) Representative: von Kreisler, Alek, Dipl.-Chem. et al,
Deichmannhaus am Hauptbahnhof
D-5000 Köln 1(DE)

## (54) Chromogenic tracers for use in an assay.

(57) A chromogen, in particular a fluorescent compound, is conjugated to a ligand through a radical derived from a dicarboxylic acid. The tracers are particularly suitable for use in a flow through assay for low molecular weight analytes. The tracers are rapidly bound, of good sensitivity, and low non-specific binding.

EP 0 132 812 A1

Croydon Printing Company Ltd.

0132812

84 108 643.2

Becton Dickinson and Company

# CHROMOGENIC TRACERS FOR USE IN AN ASSAY

The present invention relates to improved compositions for use in nonisotopic assays for the determination of haptens, antigens and antibodies (hereinafter referred to as "analytes") in liquid samples, including body fluids such as serum, sputum, urine and cerebral spinal fluid. The invention is particularly directed to fluorescent assays and to a method for preparing such conjugates and an improved assay method and test kit.

Immunoassay methods, in general, are based on the competition between a specific analyte, the amount of which is to be determined in the sample, and a known amount of the analyte or appropriate analog thereof in labeled form (tracer) for a limited number of available binding sites on a binder which is specific towards the analyte and tracer. Thus, in a system consisting of an unknown amount of analyte, a known amount of a tracer and a limited known amount of binder, the greater the concentration of analyte in the sample, the less the tracer will be bound by the binder.

If the concentration of tracer and binder is fixed and the only variable is the level of analyte, it is possible to establish an assay system for measuring the unknown level of analyte, by determining the amount of bound and/or free tracer in the system. Commonly used labels include radioisotopes, fluorescent dyes and enzymes. The activity of the radioisotope, the fluorescent intensity of the dye or the activity of the enzyme on a substrate is compared with the values given by a range of known amounts of the analyte treated in the same manner. The values obtained from the determination of the standard samples are used for establishing a standard calibration curve for the specific system and this curve is then used to determine an unknown concentration of the analyte in an unknown sample.

Various automatic flow through systems are also available for using labeled and unlabeled ligands. A flow through system wherein the tracer is bound to an adsorbent in a column and is elutriated in a differential pattern can be used for establishing standard calibration curves for specific systems and these curves are then used to determine an unknown concentration of analyte in a sample.

Immunoassay is a field where sensitivity is of prime importance due to the low analyte levels that are measured. Radioimmunoassay has been widely used due to the relative ease with wich a ligand can be labeled with a radioisotope. Radioimmunoassay sensitivity is limited, however, to about $10^{-12}$M and is more often only in the $10^{-8}$ to $10^{-10}$M range. In addition, radiolabels suffer from the drawback of short halflife and handling hazards. There is a trend in the immunoassay field to develop nonisotopic immunoassay methods.

The sensitivity of fluorescence assay, although theoretically very high, is often limited by the presence of background fluorescence. In many situations, it is impossible to reduce the background sufficiently to obtain the desired sensitivity. The present invention is directed to providing fluorescent conjugates which are highly sensitive and which resolve many problems which have been faced by the use of fluorescence assays.

U.S. Patent No. 4,268,663 describes macromolecular compositions which are used as glycosidase substrates. The glycosidase substrates employ a spacer arm to link a glycosidyl substituted chromophore to a macromolecular hub. The spacer arm is used to provide steric exclusion for modulating a signal in relation to the amount of analyte in an assay medium.

U.S. Patent No. 4,329,461 to Khanna discloses fluorescent thyroid hormone conjugates of the formula

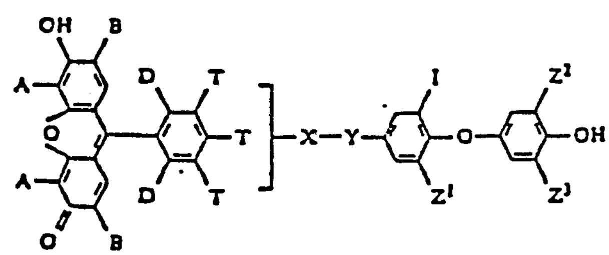

wherein:
A is any group which does not detrimentally affect the fluorescence efficiency of physical characteristics of the compound, particularly water solubility; A will normally be such groups as hydrogen, alkyl, halo, particularly of atomic number 9 to 53, more particularly of atomic number 17 to 35, and subsituted alkyl, where

alkyl may be substituted with halo as described previously, chalcogen of atomic number 8 to 16 (oxygen and sulfur) as hydroxy, mercapto, oxyether of from 1 to 2 carbon atoms, or thioether of from 1 to 2 carbon atoms, non-oxocarbonyl, particularly carboxy, alkoxycarbonyl, having alkoxy of from 1 to 2 carbon atoms, or carboxamide, the total number of substituents being normally of from 1 to 4, more usually from 1 to 2, wherein the alkyl groups are from 1 to 6, more usually from 1 to 2 carbon atoms.

In accordance with one aspect of the present invention, there is provided a conjugate comprised of a chromogen linked to a ligand through a defined spacer group to provide a tracer for use in an assay.

In accordance with another aspect of the invention, there is provided an assay for an analyte in which the tracer is a conjugate comprised of a chromogen linked to a ligand through a defined spacer group.

In accordance with a further aspect of the invention, there is provided an assay kit for use in an assay for an analyte which kit includes as a tracer a conjugate of a chromogen linked to a ligand through a defined spacer group.

More particularly, the tracer is comprised of a chromogen linked or conjugated to a ligand through a spacer radical derived from a dicarboxylic acid.

The spacer radical is represented by one of the following structural formulas:

(a) $-\overset{O}{\overset{\|}{C}}-R-\overset{O}{\overset{\|}{C}}-$

(b) $-\overset{O}{\overset{\|}{C}}-R-\overset{O}{\overset{\|}{C}}-NHNH-$

(c) $-NH-NH-\overset{O}{\overset{\|}{C}}-R-\overset{O}{\overset{\|}{C}}-NHNH-$

wherein R is a substituted or unsubstituted divalent aliphatic hydrocarbon radical having from 1 (preferably at least 2) to 13 carbon atoms, and when R is substituted, the substituent group is preferably a hydrophylic group such as hydroxyl, carboxy, amino or thio.

The tracers produced in accordance with the present invention are represented by the following structural formulas:

(1) $A-\overset{O}{\overset{\|}{C}}-R-\overset{O}{\overset{\|}{C}}-B$

(2) $A-NH-NH-\overset{O}{\overset{\|}{C}}-R-\overset{O}{\overset{\|}{C}}-B$

(3) $A-NH-NH-\overset{O}{\overset{\|}{C}}-R-\overset{O}{\overset{\|}{C}}-NHNH-B$

wherein R is as hereinabvoe defined and A is one of the chromogen and ligand and B is the other of the chromogen or ligand.

In producing the conjugate, in the case where the spacer radical is conjugated through its carbonyl group, the chromogen or ligand, which is conjugated thereto, has a substituent group capable of reacting with the carbonyl group to form the conjugate; in particular, an amino or hydroxyl group. Similarly, in the case where the spacer radical is conjugated through its hydrazide group, the chromogen or ligand, which is conjugated thereto, has a substituent group capable of reacting with the hydrazide group; in particular, a carbonyl group.

Thus, the tracer is comprised of a chromogen, preferably a fluorescent compound, which functions as a marker, which is conjugated to a ligand through a spacer radical of the type hereinabove described.

As used herein, the term "analyte" refers to a hapten, antigen oder antibody to be assayed.

As used herein, the term "ligand" refers to a hapten, antigen or antibody. In an assay for an analyte, the ligand, which is a portion of the conjugate used as a tracer, depending on the analyte, is either the analyte, an appropriate analog of the analyte, or a binder for the analyte.

As used herein, the term "appropriate analog" refers to an analog of the analyte which is bound by the binder used in the assay for the analyte.

The chromogen which is used as the identifiable marker for the tracer is an absorbing dye and preferably an absorbing dye which fluoresces (fluorescent compound). It is also possible to use a non-fluorescing chromogen. The fluorescent compound includes or is provided with a substituent group which is capable of being linked to the carboxyl group of the spacer radical for producing a conjugate with the ligand. The chromogens are usually aromatic compounds which may be fused or non-fused. As representative classes of fluorescent compounds there may be mentioned, for example, fluoresceins, rhodamines, coumarins, amino naphthalene derivatives (dansyl compounds), carbocyanins, indoles, lathanide chelates, etc. Of particular interest for use as fluorescent markers as a result of their sensitivity are fluorescein dyes, particularly fluorescein amine.

The selection of a suitable fluorescent marker for conjugation to a ligand in accordance with the invention is deemed to be within the scope of those skilled in the art from the teachings herein.

In producing a tracer in accordance with the present invention, R preferably has no more than 6 carbon atoms, and is most preferably alkylene. Particularly preferred results are obtained when the spacer radical is derived from succinic acid or succinic anhydride, i.e., R is $-CH_2-CH_2-$.

The conjugate comprised of a chromogen linked to a ligand through a spacer radical derived from a dicarboxylic acid, of the type hereinabove described, may be produced by linking or conjugating the spacer radical to both the fluorescent compound and the ligand by any one of a wide variety of procedures, which are known in the art, for producing linkages of the type hereinabove described.

Thus, for example, peptide linkages may be formed by an active ester technique. Since these procedures are well known in the art, no further details in this respect are deemed necessary for a complete understanding of the present invention.

The tracers of the present invention may be employed in an assay for any one of a wide variety of analytes. The analytes, which are preferably assayed in accordance with the invention, are those having a molecular weight up to about 6,000, with the present invention being particularly suitable for analytes having a molecular weight up to about 2,000. Thus, the invention has particular applicability to assays for low molecular weight hormones and drugs, with particularly good results being obtained in an assay for digoxin.

The binders used in the assay, in the case where the analyte is a hapten or antigen, may be an antibody or naturally occurring substance which has one or more binding sites specific for the analyte, and in the case where the analyte is an antibody, the binder may by an antigen to the antibody or an antibody elicited in response to the analyte. The selection of a suitable binder is deemed to be within the scope of those skilled in the art, and no further details in this respect are deemed necessary for a complete understanding of the invention.

In the assay, the ligand portion of the tracer used in the assay is determined by the type of assay to be employed. Thus, for example, if the assay is for an analyte which is either an antigen or hapten, the ligand portion of the tracer is either the antigen or hapten to be assayed or appropriate analog thereof.

Alternatively, the ligand portion of the tracer may be a binder for the hapten or antigen to be assayed, in which case the assay is designed so that the analyte inhibits binding of the tracer to binding sites specific for the tracer.

In the case where the analyte is an antibody, the ligand portion of the tracer may be the antibody or appropriate analog thereof, in which case both the antibody and the tracer would compete for limited number of binding sites specific for both the antibody analyte and the tracer. Alternatively, the ligand portion of the tracer may be an antigen to the antibody analyte or antibody elicited in response to the antibody analyte, in which case, the antibocy analyte inhibits binding of the tracer to binding sites specific for the tracer.

In some cases, where the analyte is to be determined by a so-called "sandwich type" of assay, the ligand portion of the tracer has binding sites specific for the analyte, which analyte has multiple determinant sites.

The selection of a suitable ligand for use as the ligand portion of the tracer is deemed to be within the scope of those skilled in the art from the teachings herein an, accordingly, no further details in this respect are deemed necessary for a complete understanding of the present invention.

The tracers of the present invention may be employed in any one of a wide variety of assays wherein it is possible to employ a chromogen, and in particular a fluorescent compound, as a marker. Thus, for example,

a tracer may be employed in a solid phase assay, a flow through type of assay, or a homogeneous assay, with the tracer being particularly suitable for use in a flow through type of assay.

As known in the art, in one type of flow through assay, the analyte and tracer (together or sequentially) are caused to flow through a chamber, which includes a binder for the tracer supported on a solid support, whereby the tracer and analyte compete for binding sites. In another type of flow through assay, the tracer and analyte are initially contacted with a binder for the analyte and tracer, and the resulting mixture, which includes analyte and tracer bound to the binder, as well as free analyte and tracer, are caused to flow through a chamber containing a separating agent, such as an adsorbent or binder (e.g. antibody) for the tracer, supported on a solid support, whereby in the chamber the free tracer is bound or adsorbed by the separating agent and the tracer previously bound to the binder passes through the chamber.

In either case, the tracer which remains in the chamber and/or the tracer which passes through the chamber is employed as a measure of the amount of analyte in the sample being assayed.

In such flow through assays, the free tracer is in contact with the binder or separating agent in the chamber for only a short period, and applicant has found that the tracers of the present invention can be effectively used in such flow through assays; i.e., the tracer is rapidly bound to the binder and/or separating agent in the chamber.

Although the tracers are particularly suitable for use in a flow through assay, the tracers are suitable for other assays, as hereinabove described. The tracers offer the further advantage, for both flow through and other types of assays, that non-specific binding is reduced and that adverse quenching of the fluorescent portion of the tracer is avoided while maintaining binding ability of the ligand portion of the tracer. In addition, the tracer is easily prepared and has good sensitivity.

As hereinabove indicated, in accordance with the preferred embodiment of the invention, there are provided tracers for use in an assay for digoxin, and the following is a preferred tracer for use in such assay.

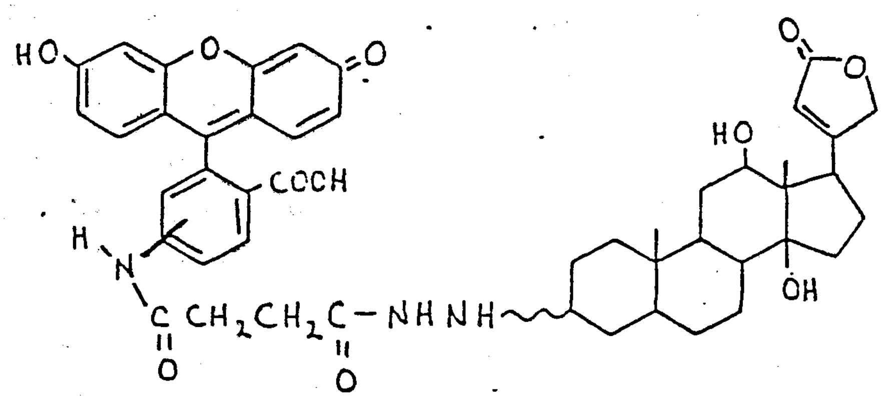

In a flow through assay employed, for example, for digoxin, the sample suspected of containing digoxin is incubated with a binder for digoxin (antibody) and a tracer in accordance with the present invention whereby the tracer and digoxin analyte compete for binding sites on the antibody. Subsequently, the mixture is caused to flow through a chamber in which digoxin antibody is supported on a solid support whereby previously bound tracer passed through the chamber and unbound tracer is bound by the supported antibody in the chamber. The amount of tracer which is bound in the chamber is directly proportional to the amount of digoxin analyte in the sample. Accordingly, the amount of digoxin analyte in the sample can be measured by determining the amount of tracer which passes through the chamber and/or which is bound to antibody in the chamber and comparing such values to those obtained with samples containing a known amount of digoxin analyte.

Although an assay procedure has been described with reference to a digoxin assay, the invention is not limited to such assays. Similarly, although a preferred embodiment has been described with respect to a flow through type of assay in which the flow through chamber includes a binder for separating bound and free components, the invention is not limited to such an assay. For example, the present invention is also applicable to a flow through type of assay in which the binder, for example, antibody, is supported on a solid support in a flow through chamber.

Similarly, the present invention is applicable to a $T_4$ assay by use of a tracer of the present invention, e.g., the following tracer:

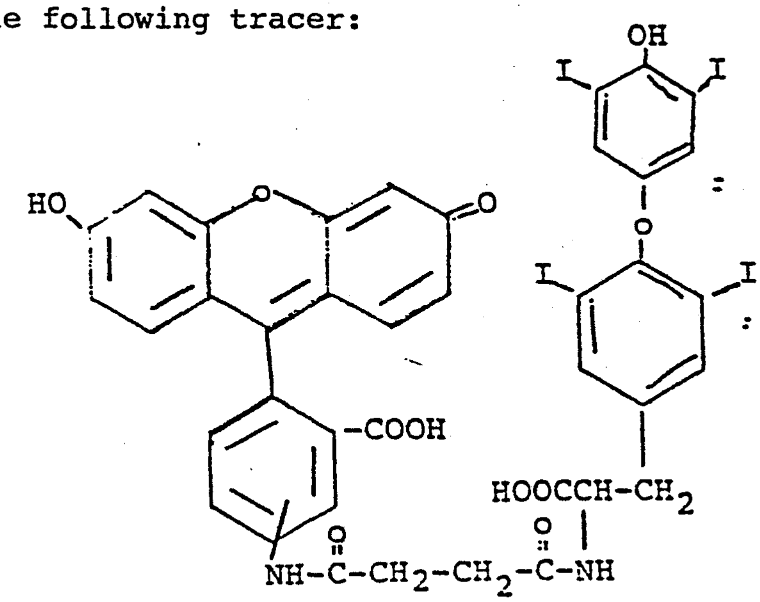

It is also to be understood that although an embodiment has been described with respect to a flow through type of assay, the present invention is also applicable to other types of assays, such as homogeneous assays, solid phase assays, and the like.

In accordance with another aspect of the present invention, there is provided a reagent kit or package for accomplishing an assay using a tracer of the present invention. The kit would include as specific components, a tracer of the type hereinabove described, as well as a binder, in particular an antibody, for the analyte to be assayed, with such binder being present either supported on a solid support or in unsupported form. The kit components, where applicable, are included in the reagent kit or package in separate containers; for example, vials. The kit may also include other components such as standards of the analyte (analyte samples having known concentrations of the analyte; buffers; and the like).

The invention will be further described with respect to the following examples; however, the scope of the present invention is not to be limited thereby:

Unless otherwise indicated, all of the temperatures are in centigrade, all percents and parts are by weight, except for mixtures of liquids, which are by volume. The following abbreviations are employed:

| | |
|---|---|
| m mol = millimole | u = micron |
| ml = milliter | cm = centimeter |
| g = gram | mm = millimeter |
| tlc = thin layer chromatography | mg = milligram |
| $R_f$ = rate of flow | N = normality |
| M = molarity | Å = angstrom |

t-BOC = tertiary butyloxycarbonyl
FITC = fluorescein isothiocyanate
DTAF = dichlorotrizinylaminofluorescein
MeOH = methanol
Si gel = silica gel
EtOAc = ethyl acetate
$CHCl_3$ = chloroform
$CH_2Cl_2$ = dichloromethane
THF = tetrahydrofuran
HOAc = acetic acid
$MgSO_4$ = magnesium sulfate
$Na_2SO_4$ = sodium sulfate
$NaHCO_3$ = sodium bicarbonate
$H_2O$ = water

## EXAMPLE 1

### FLUORESCEINAMINE-DIGOXIGENIN CONJUGATE

Fluoresceinamine (isomer II) (3.47 g, 10.0 m mol) was dissolved in 50 ml of dry pyridine and the solution cooled to 0°C. To the rapidly stirred solution was added 3.01 g (20.0 m mol) of 3-carbomethyoxypropionyl chloride and after 1.0 hr, 1.01 g (10.0 m mol) of triethylamine was added. An addition of 1.51 g (10.0 m mol) of the acid chloride was made 2.0 hr following the initial addition, and the resulting mixture was allowed to stand overnight. The reaction mixture was evaporated and the residue treated with 150 ml 1 M HCl and 150 ml of EtOAc. The organic phase was separated, washed with brine, dried ($Na_2SO_4$) and evaporated. The crude product (I) was used without further purification.

The crude ester (I) was dissolved in 70 ml of MeOH, and a solution of 25 ml of hydrazine hydrate in 50 ml

of MeOH was added with stirring. The reaction mixture was allowed to stir at room temperature for 1.0 hr, and the volatiles were removed by evaporation. The residue that remained was dissolved in water, and citric acid was added to precipitate the product. The resulting suspension was extracted with EtOAc, the layers separated, the EtOAc layer extracted with water, filtered through Whatman PS-1 filter paper, and evaporated to give the crude product. This material was takenup in MeOH, a small amount of silica gel added, and the solvent evaporated. The silica-absorbed product was chromatographed on a silica gel column eluted initially with 5 % MeOH/$CHCl_3$. Eluents were increased in polarity (% MeOH) until product was eluted. The relatively pure hydrazide derivative was isolated as red-orange powder having an $R_f$ (silica gel) of 0.14 when developed with 20 % MeOH/$CHCl_3$.

The hydrazide (II) (30.6 mg, 0,0700 m mol) and 3-keto-digoxigenin (11.5 mg, 0.0300 m mol) were dissloved in 1.0 ml of MeOH and the reaction mixture allowed to stand for 1.0 hr. Half of the reaction solution was then treated with an excess of solid sodium cyanoborohydride and the reaction mixture allowed to stand for several days. Following evaporation, the residue was purified by preparative-layer chromatography on a 20 x 20 cm silica gel plate (1.0 mm, Analtech, 20 % MeOH/$CHCl_3$). The major band was isolated and rechromatographed on five 20 x 20 cm, silica gel, analytical plates (EM, 20 % MeOH/$CHCl_3$) to provide 1.45 mg of a high $R_f$ component and 1.90 mg of a lower $R_f$ component. The conjugate of higher $R_f$ showed significant binding in the bioassay test system. The $R_f$ of both bands run over a 5.5 cm distance was identical: 0.55 (developed in 20 % MeOH/$CHCl_3$).

A $T_4$ tracer having hereinabove structural formula II may be prepared from the ester (I) of Example 1 by converting the carboxyl moiety to an active ester form, followed by reaction with L-thyroxine employing procedures known in the art.

The digoxin tracer of Example 1 may be employed in an assay as described in the following Example 2. The assay is described with reference to the automated instrument sold by Becton Dickinson Immunodiagnostics under the ARIA mark.

## EXAMPLE 2

## DIGOXIN ASSAY

I Reagents

1. Adsorption Buffer #5, pH 7.4.
   ARIA reagent - Catalog No. 614335. Contains phosphate, sodium chloride, and preservative.
2. Elution Buffer #4, pH 10.2
   ARIA reagent - Catalog No. 614211P. Contains 50 % methyl alcohol, glycine buffer, and preservative.
3. Digoxin Standards.
   ARIA reagent - Catalog No. 612839. Contains digoxin in stabilized human plasma with preservatives.
4. Digoxin Antibody Chamber.
   ARIA reagent - Catalog No. 630519. Contains digoxin antibody covalently bonded to cellulose.
5. Digoxin Fluorescent Tracer (Tracer of Example 1).
   Diluted in Adsorption Buffer #5, pH 7.4 (Reagent #1).
6. Digoxin Polyclonal Antibody.
   Diluted in Adsorption Buffer #5, pH 7.4 (Reagent #1).
7. Distilled water.

II. Instrument Setup

1. Insert DISC 2 into disc reader. Read File 27 into the computer.
2. Place reagent bottles containing sufficient volumes for the entire run on the instrument as follows:

   Blue Cap - Adsorption buffer #5, pH 7.4 (Reagent #1).
   Yellow Cap - Distilled water (Reagent #7).
   Red Cap - Elution buffer #4, pH 10.2. (Reagent #2).
   Green Ring - Digoxin fluorescent tracer (Reagent #5).
   Brown Ring - Digoxin polyclonal antibody (Reagent #6).

3. Ensure that the Beta/Gamma valve is pulled out into the GAMMA position.
4. Ensure that the G-H valve is pulled out to the H position.
5. If this is the first digoxin run, insert two priming blanks into the antibody and prefilter positions, and perform priming routine (OP CODE 9).
6. After priming has been completed, remove priming blanks, and attach two antibody chambers to the antibody and the prefilter positions.
7. Pipet 125 ul of standards controls and patient samples into sample cups and cap with cone-shaped caps.
8. Load carousel with sample cups as follows:

Inner Ring:

| Position No. | Contents |
|---|---|
| *101 | 350 ul zero standard |
| 102, 103 | adsorption buffer #5 |
| 104-106 | zero standard |
| 107, 108 | 0.5 mg/ml std |
| 109, 110 | 1.0 mg/ml std |
| 111, 112 | 2.0 mg/ml std |
| 113, 114 | 4.0 mg/ml std |
| 115, 116 | 8.0 mg/ml std |
| 118- | patient samples |
| ** last ten cups of run | adsorption buffer #5 |

Outer Ring:

Place sufficient number of empty reaction cups for entire assay.

* Once tracer, sample, antibody, and buffer have been pipetted into the reaction cup, the run is aborted, the reaction cup removed, saved, and replaced with an empty reaction cup. This is repeated again. The third time the machine is started, the run continues uninterrupted. Once buffer from position 102 and 103 are transferred to the reaction cups, the reaction cups are replaced with the reaction cups prepared by the machine initially giving three maximum binding samples.

** The last ten buffer cups are used in the assay to eliminate the gather phase of the program. The first two are replaced with reaction cups containing 100 ul standard and 555 ul adsorption #5 which will be used for serum blanks.

III. Programming

1. Go through OP CODE 20 for new reagents.
2. Select OP CODE 13; begin with number 101 and end with the final assay sample number.
3. The final sample number may be changed during the assay by utilizing BREAK-BREAK-2-3.
4. The assay can be aborted by utilizing BREAK-BREAK-2-3.

IV. Sample Collection

A fraction collector is hooked up to the ARIA so that all reagents passing through the antibody chamber are collected as follows:

| Tube # | Contents |
| --- | --- |
| 1. | 5.4 ml of buffer |
| *2. | 950 ul of eluted sample |
| 3. | 905 ul of elution buffer |

* Tube 2 is taken, vortexed, and read on the fluorometer.

V. Fluorescence Determination - PE 650

A. Instrument Settings
Excitation = 488 nm slit = 10
Emission = 515 nm slit = 15

B. Calibrate 950 ul volume using the time (seconds) dial on the controller. Fill about 5 tubes with 950 ul glycine methanol buffer, sample, and aspirate long enough to get entire sample through the cuvette without drawing air bubbles (vacuum gauge should be at 17 psi).

C. With time delay at 60 seconds, aspirate the first maximum binding tube, and set instrument so meter reads approximately 1200.

D. Set time delay at 30 seconds and read remainder of tubes.

## VI. Calculations

A. Substract each value from the average maximum binding value. This will give the net fluorescence equivalent to the first bound (or soluble bound) fraction.

B. Divide each value from A. by the average net fluorescence of the zero standards to give you the $B/B_o$ values.

C. Plat the $B/B_o$ values for the standards on log/logit paper, and calculate the concentration of digoxin in the unknowns.

## VII. Notes

1. Total tubes are prepared by taking 94.7 ul of the tracer dilution diluted to 950 ul with glycine methanol buffer. These are used to determine maximum column binding:

$$\frac{Fl_{Max.Bind} - Fl_{S.B.}}{Fl_{Total} - Fl_{G/M}} = \text{Column Binding}$$

2. To determine percent binding of soluble antibody:

$$\frac{Net_{Fluor}\ \text{of Zero (1st Bound)}}{Fl_{Max.Bind.} - Fl_{S.B.}}$$

Numerous modifications and variations of the present invention are possible in light of the above teachings, and, therefore, within the scope of the appended claims, the invention may be practiced otherwise than as particularly described.

WHAT IS CLAIMED IS:

1. A tracer for use in an assay comprising: a chromogen conjugated to a ligand through a spacer radical, said spacer radical for conjugating the chromogen to the ligand being derived from a dicarboxylic acid and selected from the group consisting of radicals having the following structural formulas:

(a) $-\overset{\overset{O}{\|}}{C}-R-\overset{\overset{O}{\|}}{C}-$

(b) $-NH-NH-\overset{\overset{O}{\|}}{C}-R-\overset{\overset{O}{\|}}{C}-$

(c) $-NH-NH-\overset{\overset{O}{\|}}{C}-R-\overset{\overset{O}{\|}}{C}-NH-NH-$

wherein R is selected from the group consisting of subsituted and unsubstituted divalent aliphatic hydrocarbon radicals having no more than 13 carbon atoms.

2. The tracer of Claim 1 wherein the chromogen is a fluorescein dye.

3. The tracer of Claim 2 wherein R is an alkylene group having 2 carbon atoms.

4. The tracer of Claim 1 wherein the tracer has the following structural formula

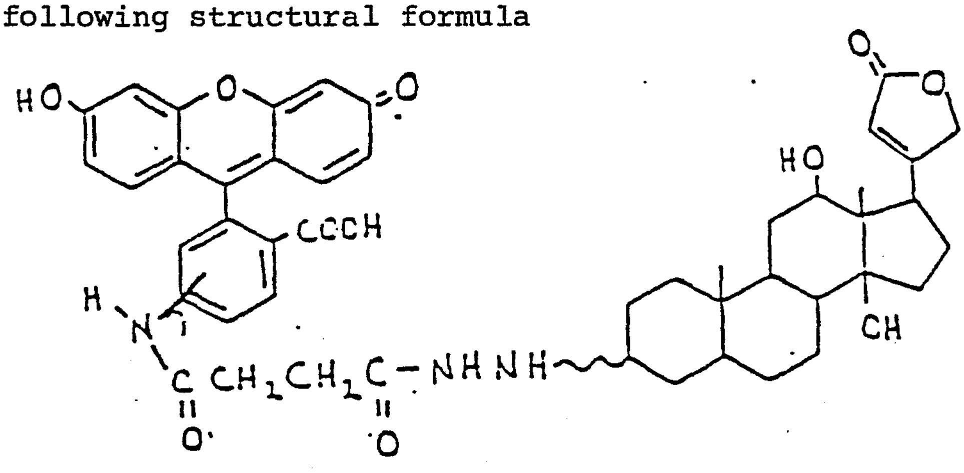

5. The tracer of Claim 1 wherein the ligand is digoxigenin.

6. The tracer of Claim 1 wherein the ligand is L-thyroxine.

7. The tracer of Claim 1 wherein the tracer has the following structural formula:

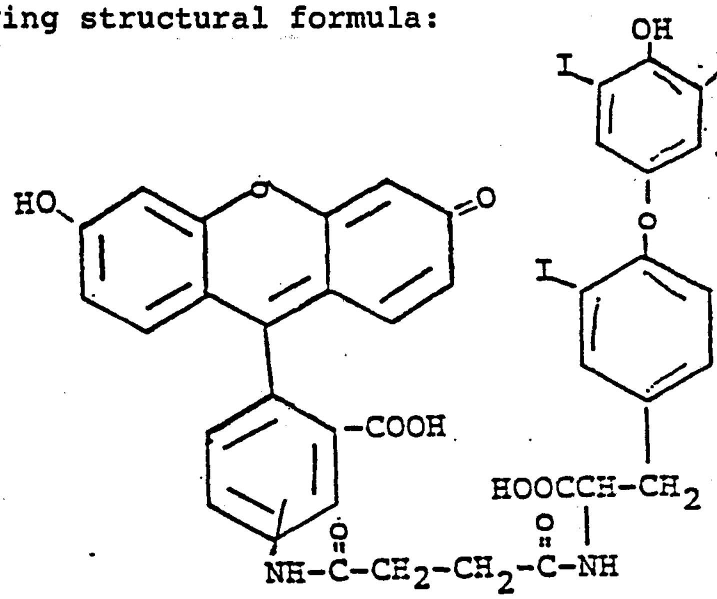

8. In an assay for an analyte employing a tracer wherein a chromogen is conjugated to a ligand through a spacer radical, the improvement comprising: said tracer being a tracer as defined in Claim 1.

9. The assay of Claim 8 wherein during the assay the tracer is passed through a flow through chamber including a binder for the tracer.

10. The assay of Claim 9 wherein the chromogen is a fluorescein dye.

11. The assay of Claim 10 wherein R is an alkylene group having 2 carbon atoms.

12. The assay of Claim 9 wherein the tracer has the following structural formula:

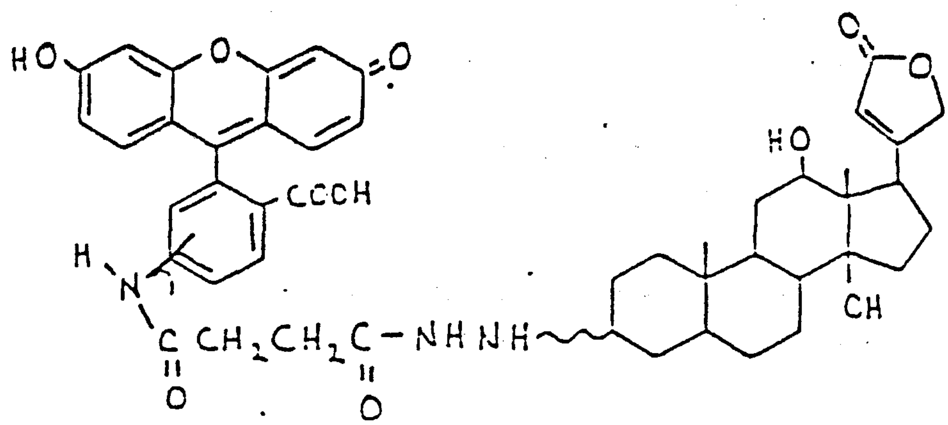

13. The assay of Claim 9 wherein the liquid is digoxigenin.

14. The assay of Claim 9 wherein the ligand is L-thyroxine.

15. The assay of Claim 9 wherein the tracer has the following structural formula:

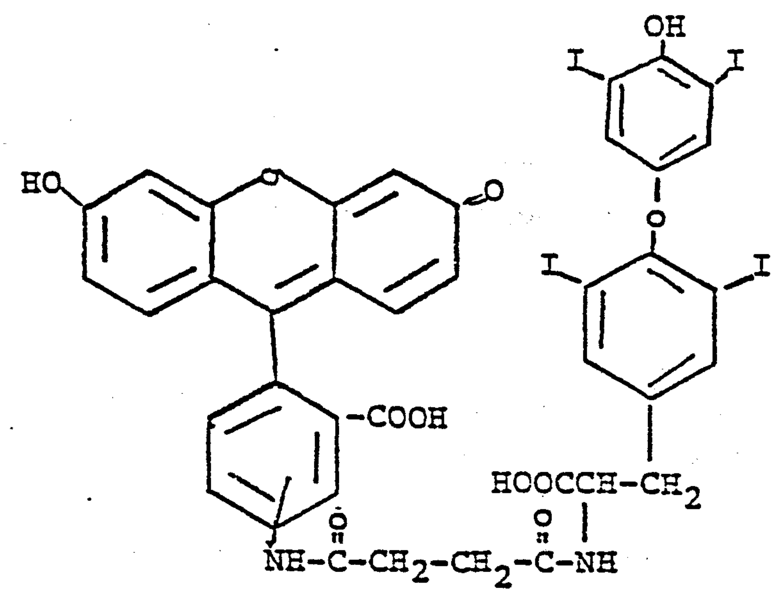

16. The assay of Claim 8 wherein the tracer has the following structural formula:

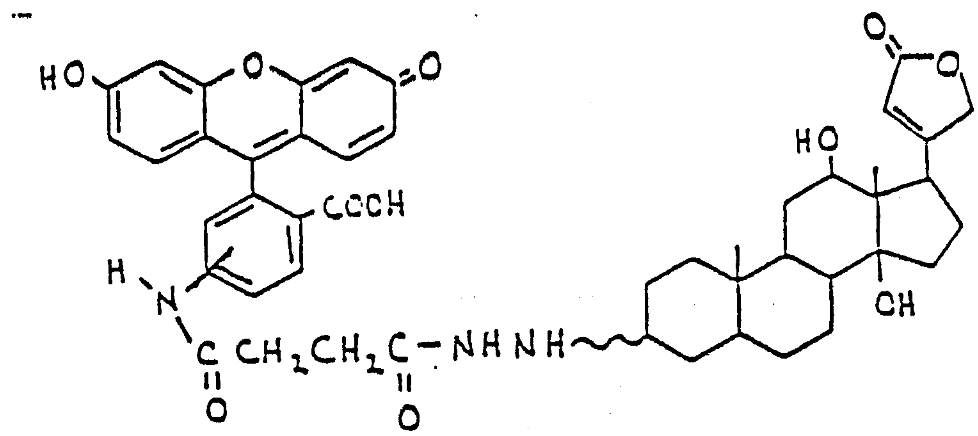

17. In a reagent kit for use in an assay for an analyte which includes a tracer wherein a chromogen is linked to a ligand through a spacer radical, and a binder for the tracer, the improvement comprising:

said tracer being a tracer as defined in Claim 1.

18. In a reagent kit for use in an assay for an analyte which includes a tracer wherein a chromogen is linked to a ligand through a spacer radical, and a binder for the tracer, the improvement comprising:

said tracer being a tracer as defined in Claim 5.

0132812

European Patent Office

# EUROPEAN SEARCH REPORT

Application number: EP 84108643.2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | GB - A - 2 012 754 (INTERNATIONAL DIAGNOSTIC TECHNOLOGY)<br>* Abstract *<br>-- | 1,2,7 | G 01 N 33/533 |
| D,A | US - A - 4 329 461 (KHANNA et al.)<br>* Abstract; claim 1 *<br>-- | 1,2,7 | |
| A | GB - A - 1 348 935 (ORGANON LABORATORIES LIMITED)<br>* Col. 5, 6; example V *<br>---- | 1,4,5 | |

TECHNICAL FIELDS SEARCHED (Int. Cl.4)

G 01 N 33/00
C 07 G

The present search report has been drawn up for all claims

| Place of search | Date of completion of the search | Examiner |
|---|---|---|
| VIENNA | 31-10-1984 | SCHNASS |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document